# EUROPEAN PATENT APPLICATION

(11) **EP 4 266 048 A1**
(43) Date of publication of application: **25.10.2023**
(21) Application number: 23162800.9
(22) Date of filing: 20.03.2023
(51) Int. Cl.: G01N 33/00, G01N 33/497, G06N 3/09, G06N 20/20

(54) **ODOR DETECTING METHOD FOR REFRIGERATING APPLIANCE AND REFRIGERATING APPLIANCE**

(30) Priority: 20.04.2022 CN 202210416972
(71) Applicant: BSH Hausgeräte GmbH, 81739 München (DE)
(72) Inventor: He, Ziyue, NANJING, 210046 (CN)

(57) **Abstract**

An odor detecting method for a refrigerating appliance and a refrigerating appliance are provided. The method includes: acquiring environmental data of gas in a refrigerating appliance (S11); inputting the environmental data to N preset machine learning models (S12) to obtain N odor detecting results; and determining odor properties of the gas in the refrigerating appliance according to the odor properties in the N odor detecting results (S13). The above solution can improve accuracy of determining the odor properties of the gas in the refrigerating appliance.

## Description

### TECHNICAL FIELD

Embodiments of the present invention relate to the technical field of refrigerating appliances, and in particular, to an odor detecting method for a refrigerating appliance and a refrigerating appliance.

### BACKGROUND

In daily life, people use a refrigerating appliance such as a refrigerator to store perishable foods such as vegetables and fish. The refrigerating appliance extends the shelf life of food by forming a low-temperature confined space. Since the food itself emits different odors, the food placed in the refrigerating appliance inevitably deteriorates and produces peculiar smells over time. These peculiar smells will have a negative impact on user experience, but the peculiar smells in the refrigeration appliance cannot be accurately detected in the prior art.

### SUMMARY

Embodiments of the present invention are intended to provide an improved refrigerating appliance and an odor detecting method for a refrigerating appliance.

Therefore, an embodiment of the present invention provides an odor detecting method for a refrigerating appliance. The method includes: acquiring environmental data of gas in a refrigerating appliance; inputting the environmental data to N preset machine learning models to obtain N odor detecting results, where the odor detecting results include odor properties, and training data adopted during training of the N preset machine learning models is obtained in the following manner: respectively performing Q rounds of odor detecting experiments for M groups of ingredients, where Q pieces of sample data are obtained for each group of ingredients and M*Q pieces of sample data are obtained in total; and combining the M*Q pieces of sample data according to a principle that a training set includes at least sample data corresponding to the M group of ingredients and the testing set includes sample data corresponding to the M group of ingredients, to obtain at least N sets of training data, where each set of training data is trained to obtain one preset machine learning model to obtain the N preset machine learning models, the training data includes the training set and the testing set, and N, M, and Q are all positive integers greater than 1; and determining odor properties of the gas in the refrigerating appliance according to the odor properties in the N odor detecting results.

Compared with the prior art, the technical solutions of the embodiments of the present invention have the following beneficial effects.

The N odor detecting results are obtained by inputting the acquired environmental data of the gas in the refrigerating appliance into the N preset machine learning models for the odor detection. The accuracy of the determined odor properties can be improved by determining the odor properties of the gas in the refrigerating appliance according to the odor properties in the N odor detecting results. Moreover, training data adopted during training of the N preset machine learning models is obtained in the following manner: respectively performing Q rounds of odor detecting experiments for M groups of ingredients, where Q pieces of sample data are obtained for each group of ingredients and M*Q pieces of sample data are obtained in total; and combining the M*Q pieces of sample data according to a principle that a training set includes at least sample data corresponding to the M group of ingredients and the testing set includes sample data corresponding to the M group of ingredients, to obtain at least N sets of training data, where each set of training data is trained to obtain one preset machine learning model to obtain the N preset machine learning models, and the training data includes the training set and the testing set. Since both the training set and the testing set include at least the sample data corresponding to the M group of ingredients, each preset machine learning model has a high accuracy in predicting results of a plurality of types of ingredients, which can improve the accuracy of detecting the odor properties of the gas in the refrigerating appliance.

Optionally, the sample data includes the environmental data obtained through the odor detecting experiments and calibrated odor properties, and the odor detecting method further includes: estimating the odor properties of each group of ingredients in the testing set according to the preset machine learning model obtained by the training set when an i^{th} preset machine learning model is obtained by training an i^{th} set of training data; determining a difference between the estimated odor properties and the calibrated odor properties of each group of ingredients; and adding, for a j^{th} group of ingredients having the difference between the estimated odor properties and the calibrated odor properties exceeding a preset difference range, sample data of the j^{th} group of ingredients in the training set of the i^{th} preset machine learning model, where 1 ≤ i ≤ N, 1 ≤ j ≤ M, and i and j are both positive integers. By determining the difference between the estimated odor properties and the calibrated odor properties, sample data of the j^{th} group of ingredients in the training set of the i^{th} preset machine learning model is added for a j^{th} group of ingredients whose difference between the estimated odor properties and the calibrated odor properties exceeds a preset difference range. In this way, the accuracy of the i^{th} machine learning model obtained by retraining can be improved for the prediction results of the j^{th} group of ingredients.

Optionally, the determining odor properties of the gas in the refrigerating appliance according to the odor properties in the N odor detecting results include any of the following: using the odor property with a largest proportion as the odor property of the gas in the refrigerating appliance according to a proportion of each odor property in the N odor detecting results; taking a median of the odor properties in the N odor detecting results and using the odor property corresponding to the median as the odor property of the gas in the refrigerating appliance; and taking an average of the odor properties in the N odor detecting results, and using the odor property corresponding to the average as the odor property of the gas in the refrigerating appliance. According to the proportion of each odor property or the median of the odor properties in the N odor detecting results, the results that the N odor detecting results mostly point to can be determined. The accuracy of the determined odor properties can be improved by taking the most pointed result (that is, the odor properties with the largest proportion or the odor properties corresponding to the median) as the odor properties of the gas in the refrigerating appliance.

Optionally, the odor properties include at least pleasant and unpleasant. For example, a different odor property may be characterized by a score of a different value or a different interval band, and the score represents a level of the odor pleasantness within the refrigerating appliance.

Optionally, when the median of the odor properties in the N odor detecting results is taken, the odor property close to the median and closest to the unpleasant is used as the odor property of the gas in the refrigerating appliance if the median has no corresponding odor property; or when the average of the odor properties in the N odor detecting results is taken, the odor property close to the average and closest to the unpleasant is used as the odor property of the gas in the refrigerating appliance if the average has no corresponding odor property. In this way, a large deviation between the output odor properties and real odor feeling of a user can be avoided, which contributes to the gap between the user's odor experience, and the unpleasant can be cleaned in time when the odor properties tend to be unpleasant.

Optionally, the N preset machine learning models are respectively constructed by using different algorithms, or the N preset machine learning models are constructed by using the same algorithm, or at least two of the N preset machine learning models are constructed by using different algorithms. In this way, advantages of a plurality of algorithms can be considered comprehensively to balance the skills of the preset machine learning model obtained from the training of different algorithms, which help to improve the accuracy and stability of the odor properties of the gas in the refrigerating appliances when the odor properties of the gas in the refrigerating appliance are obtained based on the N odor detecting results.

Optionally, the algorithm includes any of the following: a multilayer neural network classifier, a K-nearest neighbor classifier, a decision tree, and a support vector machine classifier.

Optionally, at least one of the N preset machine learning models includes a plurality of sub-models, the plurality of sub-models are constructed by using different algorithms, and the odor detecting results of the preset machine learning model including the plurality of sub-models are obtained based on the odor detecting results obtained by the plurality of sub-models. The use of appropriate means for the preprocessing is conducive to realization of targeted processing according to the characteristics of each dimension data and improve rationality of abnormal data elimination.

Optionally, after the acquiring environmental data of gas in a refrigerating appliance, the odor detecting method further includes: preprocessing the environmental data, where a preprocessing method used for data in at least one dimension in the environmental data is different from a preprocessing method used for data in other dimensions.

Optionally, the preprocessing the environmental data includes: performing parameter scaling processing on the environmental data to unify the data in different dimensions into a data range of the same dimension, where a parameter scaling method used for the data in the at least one dimension is different from a parameter scaling method used for the data in the other dimensions; standardizing the processed environmental data so that the environmental data satisfies normal distribution; and extracting a feature of the processed environmental data to obtain the preprocessed environmental data. Since an initial order of magnitude of the environmental data in different dimensions differs greatly, which may affect a subsequent data processing, the parameter scaling method adapted to the dimensions is used in the implementation to compress the environmental data in each dimension to the same order of magnitude without changing the data features, so as to optimize the subsequent feature extraction effect.

Optionally, the environmental data includes gas data, temperature data, and humidity data, and the performing parameter scaling processing on the environmental data, to unify the data in different dimensions into a data range of the same dimension includes: taking a logarithm of a value of the gas data to nonlinearly compress the value range of the gas data into a preset value interval; and performing maximum minimization on the temperature data, the humidity data, and the gas data after taking the logarithm, to obtain the processed environmental data. Therefore, the parameter scaling method adapted to the dimensions is used for compressing the environmental data in each dimension to the same order of magnitude without changing the data features.

Optionally, before the standardizing the processed environmental data, the preprocessing the environmental data further includes: performing data smoothing on the processed environmental data. As a result, the noise in the environmental data can be effectively processed, so that the impact of the noise on the overall data sample is reduced. Therefore, classification of a subsequent preset classification model is more accurate.

An embodiment of the present invention further provides a refrigerating appliance, including: a gas detector, configured to collect environmental data of gas in the refrigerating appliance; and a control module, configured to communicate with the gas detector to receive the environmental data, where the control module performs the odor detecting method according to any of the above embodiments.

Optionally, the refrigerating appliance further includes a deodorization module. The control module is further configured to generate a control instruction according to a current odor property in the refrigerating appliance; and the deodorization module adjusts an operating parameter according to the control instruction in response to the received control instruction.

Further features of the present disclosure are apparent from the claims, the figures and the description of figures. The features and feature combinations mentioned above in the description as well as the features and feature combinations mentioned below in the description of figures and/or shown in the figures alone are usable not only in the respectively specified combination, but also in other combinations without departing from the scope of the invention. Thus, implementations are also to be considered as encompassed and disclosed by the invention, which are not explicitly shown in the figures and explained, but arise from and can be generated by separated feature combinations from the explained implementations. Implementations and feature combinations are also to be considered as disclosed, which thus do not comprise all of the features of an originally formulated independent claim. Moreover, implementations and feature combinations are to be considered as disclosed, in particular by the implementations set out above, which extend beyond or deviate from the feature combinations set out in the back-references of the claims.

### BRIEF DESCRIPTION OF THE DRAWINGS

FIG. 1 is a flowchart of an odor detecting method according to an embodiment of the present invention.
FIG. 2 is a flowchart of a data preprocessing method according to an embodiment of the present invention.

### DETAILED DESCRIPTION

In order to make the foregoing objectives, features, and beneficial effects of the embodiments of the present invention more apparent and easier to understand, specific embodiments of the present invention are described in detail below with reference to the accompanying drawings.

An embodiment of the present invention provides an odor detecting method applicable to an odor monitoring scene in a refrigerating appliance, for example, monitoring whether a peculiar smell exists in the refrigerating appliance, and for another example, monitoring realtime odor properties in the refrigerating appliance. The odor properties may be used for describing odor pleasantness in the refrigerating appliance, and for example, may include at least two categories: pleasant and unpleasant. In practical application, a classification standard and a quantity of categories for classification of odor properties may be adjusted as required. For example, the odor properties may be further subdivided into five categories: very pleasant, pleasant, neutral, unpleasant, and extremely unpleasant (which may also be referred to as very unpleasant).

Different odor properties may be characterized by scores of different values or different interval sections, and the scores represent levels of the odor pleasantness in the refrigerating appliance. For example, a score of 1 represents pleasant and a score of 0 represents unpleasant. For another example, scores in a range of [0,1) represent unpleasant, and scores greater than or equal to 1 represent pleasant.

Ingredients stored in the refrigerating appliance may belong to a single category or a plurality of categories. The implementation may specially detect the odor property of an odor produced by a specific type of ingredient in the refrigerating appliance, or may macroscopically detect the odor property of a mixed odor produced by a plurality of ingredients in the refrigerating appliance as a whole.

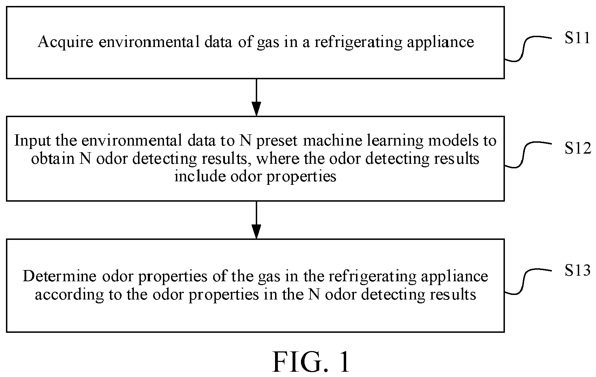

Referring to FIG. 1, FIG. 1 is a flowchart of an odor detecting method according to an embodiment of the present invention. The method may specifically include the following steps.

Step S11: Acquire environmental data of gas in a refrigerating appliance.

Step S12: Input the environmental data to N preset machine learning models to obtain N odor detecting results, where the odor detecting results include odor properties.

Step S13: Determine odor properties of the gas in the refrigerating appliance according to the odor properties in the N odor detecting results.
training data adopted during training of the N preset machine learning models is obtained in the following manner: respectively performing Q rounds of odor detecting experiments for M groups of ingredients, where Q pieces of sample data are obtained for each group of ingredients, and

M*Q pieces of sample data are obtained in total; and combining the M*Q pieces of sample data according to a principle that a training set includes at least sample data corresponding to the M group of ingredients and the testing set includes sample data corresponding to the M group of ingredients, to obtain at least N sets of training data, where each set of training data is trained to obtain one preset machine learning model to obtain the N preset machine learning models, the training data includes the training set and the testing set, and N, M, and Q are all positive integers greater than 1.

In some embodiments, the food types corresponding to M group of ingredients do not overlap. That is, the M group of ingredients are completely different. The N preset machine learning models obtained by the training are different in parameters and structures, therefore the N preset machine learning models are also different in the ability to detect odors. To determine the odor properties of the gas in the refrigerating appliance according to the odor properties in the N odor detecting results can better balance skills of different preset machine learning models, making determination results of the odor properties of the gas in the refrigerating appliance more stable and accurate.

For example, three rounds of odor detecting experiments (recorded as round 1, round 2, and round 3, respectively) are performed on three groups of ingredients A, B, and C. The sample data of the three groups of ingredients in the first round of odor detecting experiment are recorded as A1, B1, and C1, the sample data in the second round of odor detecting experiment are recorded as A2, B2, and C2, and the sample data of the third round of odor detecting experiment are recorded as A3, B3, and C3, and a total of 9 groups of sample data are obtained. Up to 27 groups of training data can be obtained by combining 9 groups of data A1, B1, C1, A2, B2, C2, A3, B3, and C3. Each group of training data includes a training set and a testing set. The training set is used for training the obtained preset machine learning model, and the testing set is used for testing the trained preset machine learning model to determine whether the trained preset machine learning model meets the requirements.

The training set described in the scenario includes a training set for building a model and further includes a verification set for optimizing the model to obtain optimal parameters.

When the 9 groups of data A1, B1, C1, A2, B2, C2, A3, B3, and C3 are combined, corresponding sample data of three groups of ingredients A, B, and C are included in the testing set. For example, the sample data of the ingredients in group A in the testing set may be A1, A2, or A3, that is, the sample data may be taken from the first, second, or third round of odor detecting experiment. Similarly, the training set also includes the corresponding sample data of the three groups of ingredients, A, B, and C.

In some embodiments, the sample data in the training set may be configured to be greater than the sample data in the testing set to ensure the training effect of the preset machine learning model. The training sets and the testing sets may be divided by permutation and combination. The training sets in the first group of training data obtained by grouping are A2, A3, B2, B3, C2, and C3, and the testing sets are A1, B1, and C1, the training sets in the second group of training data is A2, A3, B2, B3, C1, and C3, and the testing sets are A1, B1, and C2, the training sets in the third group of training data is A2, A3, B2, B3, C1, and C2, and the testing sets are A1, B1 and C3, the training sets in the fourth group of training data is A2, A3, B1, B3, C2, and C3, and the testing sets are A1, B1, and C3, the training sets in the fifth group of training data is A2, A3, B1, B3, C1, and C3, and the testing sets are A1, B1, and C2; ..., and the training sets in the twenty-seventh group of training data is A1, A2, B1, B2, C1, and C2, and the testing sets are A3, B3, and C3.

For ease of understanding, results of one piece of the training data obtained in the division manner of permutation and combination are schematically described above. In practice, sample data of any of M group of ingredients may further be added to the testing set based on the above. Correspondingly, a quantity of sets of training data obtained also changes, which is not described by using examples one by one herein.

It may be understood that when the quantity of groups of ingredients is other values and the quantity of rounds of the odor detecting experiment is other values, the obtained sample data is grouped. When each set of training data is obtained, reference may be made to the above examples, which are not described by using examples one by one herein.

It should be noted that when the amount of sample data is large enough, the sample data may be divided into the training set and the testing set at a ratio of 7:3.

In a specific implementation, estimating the odor properties of each group of ingredients in the testing set according to the preset machine learning model obtained from the training set, when an i^{th} preset machine learning model is obtained by training an i^{th} group of training data; determining a difference between the estimated odor properties and the calibrated odor properties of each group of ingredients; and adding, for a j^{th} group of ingredients having the difference between the estimated odor properties and the calibrated odor properties exceeding a preset difference range, sample data of the j^{th} group of ingredients in the training set of the i^{th} preset machine learning model, where 1 ≤ i ≤ N, 1 ≤ j ≤ M, and i and j are both positive integers.

For example, when the first group of training data is used for training the first preset machine learning model, the testing set in the first group of training data is used for testing the first preset machine learning model based on the training set. It is found that an outputted odor property of the first preset machine learning model for ingredients of group A is 0, and the calibrated odor property is 2, where 0 can represent pleasant and 2 represents extremely unpleasant. A difference between the estimated odor properties and the calibrated odor properties exceeds a preset difference range, which indicates that accuracy of the first preset machine learning model obtained by the training on an estimated result of the ingredient of group A is low. That is, less features are learned from the ingredients of group A. In this way, the sample data of the ingredients of group A in the training set of the first preset machine learning model can be increased, and the first preset machine learning model continues to be trained based on the training set after the sample data of the ingredients of group A is increased until the first preset machine learning model meeting the requirements is obtained.

The difference between the estimated odor properties and the calibrated odor properties exceeds a preset difference range may be the difference between the score of the estimated odor properties and the score of the calibrated odor properties exceeds a set threshold, or the difference between the estimated odor properties and the pleasantness degree of the calibrated odor properties exceeds a set degree level. If the pleasantness degree of the estimated odor properties is the pleasant, and the pleasantness degree of the calibrated odor properties is unpleasant, the set degree level is determined to be exceeded. Specifically, the pleasantness level may be rated from the pleasant to the unpleasant, including five levels such as extremely pleasant, pleasant, neutral, unpleasant, and extremely unpleasant, and the degree of setting can be two levels. Setting the degree level is related to how the pleasant level is graded.

In a non-limiting embodiment, the gas data may be acquired through a gas detector arranged in the refrigerating appliance. Specifically, Step S11 may include steps: adjusting an operating temperature of the gas detector during an operation of the gas detector, and receiving candidate gas data acquired by the gas detector at each of the plurality of operating temperatures, where the candidate gas data associated with a different operating temperature is used for characterizing concentrations of different gas components; and generating the gas data based on the received plurality of candidate gas data. As a result, the operating temperature of the gas detector can be adjusted within a certain temperature range to enrich the diversity of the output data of the gas detector, so as to realize the detection of a plurality of gas components.

Further, the gas detector may include a volatile organic compounds (VOC) sensor. The VOC sensor violently reacts to specific types of gas at a different temperature, which allows the gas detector used in this implementation to detect the plurality of gas components within a certain temperature range. For example, the VOC sensor reacts with a specific type of gas component in the air and outputs a resistance value at a specific operating temperature. The resistance value can be used for characterizing concentration of the particular type of gas component.

For example, different types of ingredients may be consciously placed during an experimental phase from producing the pleasant to producing extremely unpleasant, and the gas produced during this time may be acquired. And then, a gas analyzer is configured to analyze the gas component in the acquired gas, such as a main gas component that is present in the gas when the unpleasant is produced. The dynamic heating temperature of the VOC sensor is adjusted according to the analyzed gas component, so that the VOC sensor is more sensitive to the gas component. The adjusted VOC sensor is mounted to a suitable position on the refrigerating appliance to receive the gas data acquired by the VOC sensor in a practical application such as during performing step S11.

In a specific embodiment, the operating temperature of the gas detector may be adjusted according to a candidate value selected from a preset operating temperature set during the operation of the gas detector. The preset operating temperature set may include a plurality of candidate values for the operating temperature. The gas detector can accurately detect the required plurality of gas components by designing the preset operating temperature set reasonably.

Specifically, the candidate value in the preset operating temperature set may be determined according to sensitivity of the gas detector to the gas component when operating at the candidate value.

Further, a time interval at which the gas detector is switched from a current operating temperature to a next operating temperature may be determined according to the time required for a corresponding gas component detected at the current operating temperature. The required time may be preset through experiments.

For example, the gas data may be generated based on all candidate gas data received after traversing the preset operating temperature set for at least one round. Therefore, the comprehensiveness of the gas data can be ensured, so that the gas data includes information about concentrations of all types of gas components that may be present in the refrigerating appliance within a period of time.

For another example, the amount of candidate gas data generating the gas data may be less than the number of candidate values in the preset operating temperature set. Therefore, a response speed of the gas detector is faster, so that the gas data can be generated more quickly.

In a specific implementation, the candidate values in the preset operating temperature set may be used for detecting the gas components that may be generated when a plurality of ingredients are stored together in the refrigerating appliance. Correspondingly, when step S11 is performed, a candidate value is selected from a single preset operating temperature set as the operating temperature of the VOC sensor no matter what kind of ingredient is currently stored in the refrigerating appliance.

In a variation, the preset operating temperature set may be targetedly designed for different kinds of ingredients. Correspondingly, when step S11 is performed, the corresponding preset operating temperature set may be retrieved according to the type of ingredients currently stored in the refrigerating appliance, and the candidate value is selected from the preset operating temperature set as the operating temperature of the VOC sensor.

Further, when a plurality of ingredients are stored in the refrigerating appliance, the preset operating temperature sets corresponding to various ingredients may be respectively retrieved for detection. In this example, the type of ingredients currently stored in the refrigerating appliance may be obtained from user input information or may be actively detected by a sensing device such as an image sensor.

In a specific implementation, a temperature and humidity collection device may be arranged in a compartment of the refrigerating appliance to collect temperature data and humidity data in the compartment. Alternatively, the VOC sensor may be reused to collect the temperature data and the humidity data.

In a variant, the environmental data may include gas data and operating parameters of the refrigerating appliance when the gas data is obtained. The operating parameters may include a compartment temperature and a compartment humidity. For example, the operating parameters may be directly obtained from a control module of the refrigerating appliance. The environmental data required for prediction of the machine learning model may also be obtained, and the operating parameters are fixed values and are not affected by an external environment with opening and closing of a door switch of the refrigerating appliance. Therefore, the effect of using the operating parameters as one piece of the environmental data in improving the reliability of the input data is expectable.

In a specific implementation, the environmental data acquired in step S11 may include raw data received from the VOC sensor. The raw data may include the resistance value outputted by the VOC sensor over a period of time, and the temperature data and the humidity data in the refrigerating appliance at the time the resistance value is generated.

Further, the acquired environmental data can be preprocessed and step 12 can be performed based on the preprocessed environmental data.

When the environmental data is preprocessed, the preprocessing method used for data in at least one dimension of the environmental data is different from the preprocessing method used for data in other dimensions.

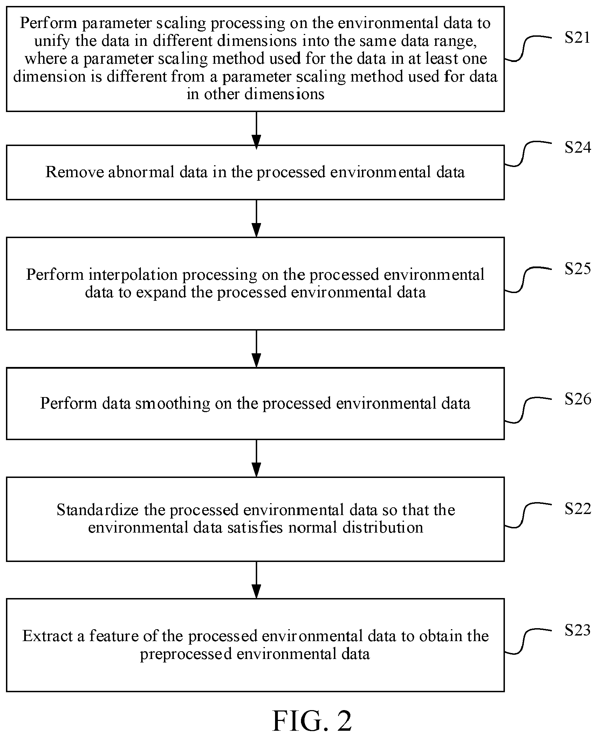

Specifically, referring to FIG. 2, FIG. 2 is a flowchart of a data preprocessing method according to an embodiment of the present invention. The method specifically includes the following steps.

Step S21: Perform parameter scaling processing on the environmental data to unify the data in different dimensions into a data range of the same dimension, where a parameter scaling method used for the data in the at least one dimension is different from a parameter scaling method used for the data in the other dimensions; and

Step S22: Standardize the processed environmental data so that the environmental data satisfies normal distribution; and

Step S23: Extract a feature of the processed environmental data to obtain the preprocessed environmental data.

Since an initial order of magnitude of the environmental data in different dimensions differs greatly, which may affect a subsequent data processing, the parameter scaling method adapted to the dimensions is used in step S21 to compress the environmental data in each dimension to the same order of magnitude without changing the data features, so as to optimize the subsequent feature extraction effect.

For example, step S21 may include steps: taking logarithmic of a value of the gas data (that is, the resistance value outputted by the VOC sensor), so as to nonlinearly compress the value range of the gas data into a preset value period; and performing a maximum minimization process on the temperature data, the humidity data, and the gas data after taking the logarithm, to obtain the processed environmental data. Therefore, the parameter scaling method adapted to the dimensions is used for compressing the environmental data in each dimension to the same order of magnitude without changing the data features.

In a practical application, the gas data may also be processed by linear compression.

The maximum minimization process is an implementation form of normalization processing, which may be set according to the detection capability of the gas detector, so that all environmental data given for the subsequent processing has a data range between 0 and 1.

In a specific implementation, after step S21 and before step S22, the preprocessing of environmental data can also include step S24: Remove abnormal data in the processed environmental data. Therefore, an impact of noise on an overall data sample is facilitated to be reduced.

For example, the abnormal data may include saturation data. The saturation data may, for example, be a resistance value that is insensitive to a change in the operating temperature. Specifically, it is found that the resistance values of some resistors are usually saturated during the experiment. For example, the resistance values are consistently detected at 102.4 M ohm, which meant that the saturated resistance values are not valid for detecting the odor changes. Therefore, the saturated resistance values can be actively discarded and removed from the environmental data when step S24 is performed.

For another example, the abnormal data may include discrete data. The discrete data may, for example, be a part of the temperature data or the humidity data of the processed environmental data whose value exceeds a temperature range or a humidity range set by the refrigerating appliance.

For still another example, the abnormal data may include incomplete data. The incomplete data may, for example, be an incomplete data set after the foregoing data cleaning. In this specific implementation, the resistance value (characterizing the gas data), the temperature data, and the humidity data are recorded as a set of data group. After elimination operations of the saturation data and the discrete data, incomplete data groups are bound exist, and these incomplete data groups are required to be eliminated. In a practical application, an initial data group outputted by the gas detector is incomplete due to a stability factor of the gas detector, therefore such data is also required to be eliminated.

In a specific implementation, after step S24 and before step S22, the preprocessing of environmental data may further include step S25: Perform interpolation processing on the processed environmental data to expand the processed environmental data. Therefore, the data sample can be effectively expanded.

For example, resistance values G2, G3, and G4 are resistance values measured at different heating time points at the same operating temperature and the difference between the resistance values significantly varies during the experiment. Therefore, in step S25, the values of G3-G2, G4-G2, and G4-G3 are added to the variable set as supplementary variables. Similarly, G6-G5, G8-G7, G9-G7, and G9-G8 are also be added to a variable set. All variables in the variable set are fed as the input data to the preset machine learning model for testing.

In a specific implementation, after step S25 and before step S22, the preprocessing of environmental data may further include step S26: Perform data smoothing on the processed environmental data. As a result, the noise in the environmental data can be effectively processed, so that the impact of the noise on the overall data sample is reduced. Therefore, classification of a subsequent preset classification model is more accurate.

The date smoothing processing can process the noise in the data, so that the impact of the noise on the overall data sample becomes less. Therefore, a subsequent training algorithm model is more accurate. In this scenario, the data smoothing method taken can take a median value of the data of a plurality of periods (including the resistance value, the temperature data, and the humidity data). Each round of odor detecting experiments can be divided into a plurality of periods, each of which collects the environmental data regularly.

After the data smoothing, part of the noise data is filtered out, fluctuation of the data is reduced, and curve becomes smoother.

In a practical application, the data smoothing processing can also be realized by averaging the data groups of the plurality of periods.

During the training of preset machine learning model, the training data used can also be preprocessed by steps S21 to S26 above.

In a specific implementation, step S13 may be achieved in either of the following ways. For example, using the odor property with a largest proportion as the odor property of the gas in the refrigerating appliance according to a proportion of each odor property in the N odor detecting results.

For another example, taking a median of the odor properties in the N odor detecting results and using the odor property corresponding to the median as the odor property of the gas in the refrigerating appliance.

According to the proportion of each odor property or the median of the odor properties in the N odor detecting results, the results that the N odor detecting results mostly point to can be determined. The accuracy of the determined odor properties can be improved by taking the most pointed result (that is, the odor properties with the largest proportion or the odor properties corresponding to the median) as the odor properties of the gas in the refrigerating appliance.

For still another example, taking an average of the odor properties of the N odor detecting results and using the odor property corresponding to the average as the odor property of the gas in the refrigerating appliance.

Further, when the median of the odor properties in the N odor detecting results is taken, the odor property close to the median and closest to the unpleasant is used as the odor property of the gas in the refrigerating appliance if the median has no corresponding odor property. In this way, the deviation between the output odor properties and real odor feeling of a user can be avoided, and the unpleasant can be detected and removed in time when the odor properties tend to be unpleasant.

Further, when the average of the odor properties in the N odor detecting results is taken, the odor property close to the average and closest to the unpleasant is used as the odor property of the gas in the refrigerating appliance if the average has no corresponding odor property. In this way, the deviation between the output odor properties and the real odor feeling of the user can be avoided, and the unpleasant can be cleaned in time when the odor properties tend to be unpleasant.

In a specific implementation, the N preset machine learning models are respectively constructed by using a different algorithm, or the N preset machine learning models are all constructed by using a same algorithm, or at least two preset machine learning models in the N preset machine learning models are constructed by using a different algorithm.

In a specific implementation, any of the following algorithms can be used for building the preset machine learning model: a K-nearest neighbor classifier (KNN), multi-layer neural network (MLP), a support vector machine classifier (SVM), a decision tree, and so on.

In a specific implementation, the odor properties in the N odor detecting results can be selected from a preset odor property set. That is to say, the environmental data is inputted into the preset machine learning model, and the odor properties obtained is one of the preset odor property set.

Specifically, the preset odor property set may include two kinds of odor properties: pleasant and unpleasant. Alternatively, the preset odor property set may include three kinds of odor properties: pleasant, unpleasant, and extremely unpleasant. Alternatively, the preset odor property set may include five kinds of odor properties: extremely pleasant, pleasant, neutral, unpleasant, and extremely unpleasant.

Three types of odor properties are used as an example. The odor properties may output a score of 0, 1, or 2. The output scores represent estimated results of the current odor properties in the refrigerating appliance based on the environmental data. 0 may represent pleasant, 1 may represent unpleasant, and 2 may represent extremely unpleasant.

In a specific implementation, the preset odor property set may include a plurality of first-level properties and a plurality of second-level properties, and the plurality of second-level properties may belong to one of the plurality of first-level properties.

Specifically, the first-level property may be, for example, the unpleasant, the pleasant, or the neutral. Similar to a multi-level directory, the second-level property may be subcategories of the first-level property. The odor monitoring scenario in the refrigerating appliance focuses on the odor property of the unpleasant. Correspondingly, a plurality of secondary properties may be set for the first-level property of the unpleasant. These second-level properties may be, for example, ordinary unpleasant and very unpleasant.

Further, a first subset may include the plurality of first-level properties, and a second subset may include the plurality of second-level properties.

For example, it is assumed that the preset odor property set = {0,1,2,3}, where 0 represents pleasant, 1 represents unpleasant, 2 represents ordinary unpleasant, and 3 represents very unpleasant, the first subset = {0,1}, and the second subset = {2,3}.

In a specific implementation, when the determined odor property is the unpleasant in the first-level properties, the unpleasant may be further subdivided to determine whether the unpleasant is ordinary unpleasant or very unpleasant, which is helpful for the refrigerating appliance to use an appropriate odor removal means to remove the peculiar smell in the refrigerating appliance.

As mentioned above, with this implementation, since the training set and the testing set of the training data used for the training of the preset machine learning model both include at least the sample data corresponding to the M groups of ingredients, each preset machine learning model has a high accuracy in predicting results of a plurality of types of ingredients. Further, the accuracy of obtaining the odor detecting result of the gas in refrigerating appliance based on the N odor detection results obtained from the N preset machine learning models is also high, therefore the accuracy of the odor detecting result of the gas in the refrigerating appliance can be improved.

An embodiment of the present invention further provides a refrigerating appliance. A gas detector is configured to acquire environmental data of gas in the refrigerating appliance; and a control module communicating with the gas detector to receive the environmental data, where the control module performs the odor detecting method according to any of the above embodiments.

Further, the refrigerating appliance may further include a deodorization module. The control module is further configured to generate a control instruction according to a current odor property in the refrigerating appliance; and the deodorization module adjusts an operating parameter according to the control instruction in response to the received control instruction. When an odor is detected based on the odor detecting method provided in the implementation, the deodorization module is actively controlled to operate and remove the odor in time.

The refrigerating appliance may include a refrigerator, a freezer, a cabinet freezer, and so on.

The gas detector can be mounted in each compartment of the refrigerating appliance to acquire the targeted environmental data of the compartment in which the gas detector is located. Alternatively, one gas detector may be arranged close to a return air outlet of the refrigerating appliance to collect the overall environmental data inside the refrigerating appliance.

The control module may include a main control board of the refrigerating appliance. Alternatively, the control module may be a module dedicated to performing the odor detecting method according to any of the above embodiments.

The deodorization module may be arranged in each compartment. For any compartment, when the prediction result of the environmental data obtained by the gas detector of the compartment by the control module indicates that the odor property of the current compartment is unpleasant, the deodorization module of the compartment operates to remove the odor of the compartment.

Alternatively, one deodorization module may be arranged in any compartment, or arranged in an air duct of the refrigerating appliance. When the odor property of any compartment is determined to be unpleasant, the deodorization module operates and realizes the overall odor removal of all compartments through a circulating refrigeration system in the refrigerating appliance.

For example, the deodorization module may include an ionizer.

Limited or wireless communication can be adopted between the gas detector and the control module, and between the control module and the deodorization module.

Further, the refrigerating appliance may include a communication module. The control module communicates with the gas detector through the communication module.

Further, the control module and the deodorization module can also communicate with each other through the communication module.

Alternatively, the communication module can communicate with the device terminal of the user to timely inform the user of the prediction result. For example, the device terminal may include a mobile phone, an IPAD, a home monitoring device, and so on.

Further, a preset classification model may be stored in the control module. Alternatively, the preset classification model may be stored in other position within the refrigerating appliance or outside the refrigerating appliance (such as in a cloud), and the control module performing the implementation can access the corresponding storage module to transmit the obtained environmental data to the module and receive the classification result of the model.

Further, the control module can preprocess the environmental data outputted by the gas detector. Alternatively, the preprocessing of the environmental data may be performed by other specialized modules within the refrigerating appliance. The module communicates with the control module to transmit the preprocessed environmental data to the control module. Alternatively, the specialized module may include the cloud. The control module uploads the received environmental data to the cloud and receives the preprocessed environmental data transmitted by the cloud.

It should be noted that each module can be independent of each other, can also be integrated in a same chip or integrated into a same functional module. For example, the control module and the communication module can be integrated together.

In the first application scenario, it is supposed that the output is "0" (pleasant) or "1" (unpleasant) according to the N preset machine learning models. Correspondingly, when the odor property of the gas in the refrigerating appliance is determined to be "1" according to the N odor detecting results of the N preset machine learning models, the control module activates the deodorization module to perform a deodorization operation. With continuous or periodic monitoring of the odor in the refrigerating appliance, the control module can turn off the deodorization module when it is determined that the odor property of the gas in the refrigerating appliance is "0".

In the first application scenario, it is supposed that the N preset machine learning models output "0" (pleasant), "1" (unpleasant), or "2" (very unpleasant). Correspondingly, when the odor property of the gas in the refrigerating appliance is determined to be "1" according to the N odor detecting results of the N preset machine learning models, the control module can control the deodorization module to operate at a lower power for the deodorization operation. When the odor property of the gas in the refrigerating appliance is determined to be "2", the control module can control the deodorization module to operate at a high power for the deodorization operation. With continuous or periodic monitoring of the odor in the refrigerating appliance, the control module can turn off the deodorization module when the output of the preset classification model is "0".

Although specific implementations have been described above, these implementations are not intended to limit the scope disclosed in the present invention, even if only a single implementation is described with respect to specific features. The feature examples provided in the disclosure of the present invention are intended for illustration but not limitation, unless otherwise stated. During specific implementation, the technical features of one or more dependent claims may be combined with the technical features of the independent claims according to the actual requirements, if technically feasible, and the technical features from the corresponding independent claims may be combined in any appropriate way, not only through the specific combination listed in the claims.

Although the present invention is disclosed as above, the present invention is not limited thereto. A person skilled in the art can make various changes and modifications without departing from the spirit and the scope of the present invention. Therefore, the protection scope of the present invention should be subject to the scope defined by the claims.

## Claims

1. An odor detecting method for a refrigerating appliance, **characterized by** comprising:
acquiring environmental data of gas in a refrigerating appliance;
inputting the environmental data to N preset machine learning models to obtain N odor detecting results, wherein the odor detecting results comprise odor properties, and training data adopted during training of the N preset machine learning models is obtained in the following manner: respectively performing Q rounds of odor detecting experiments for M groups of ingredients, wherein Q pieces of sample data are obtained for each group of ingredients and M*Q pieces of sample data are obtained in total; and combining the M*Q pieces of sample data according to a principle that a training set comprises at least sample data corresponding to the M group of ingredients and the testing set comprises sample data corresponding to the M group of ingredients, to obtain at least N sets of training data, wherein each set of training data is trained to obtain one preset machine learning model to obtain the N preset machine learning models, the training data comprises the training set and the testing set, and N, M, and Q are all positive integers greater than 1; and
determining odor properties of the gas in the refrigerating appliance according to the odor properties in the N odor detecting results.

2. The odor detecting method according to claim 1, **characterized in that** the sample data comprises the environmental data obtained through the odor detecting experiments and calibrated odor properties, and the odor detecting method further comprises:
estimating the odor properties of each group of ingredients in the testing set according to the preset machine learning model obtained by the training set when an i^{th} preset machine learning model is obtained by training an i^{th} set of training data;
determining a difference between the estimated odor properties and the calibrated odor properties of each group of ingredients; and
adding, for a j^{th} group of ingredients having the difference between the estimated odor properties and the calibrated odor properties exceeding a preset difference range, sample data of the j^{th} group of ingredients in the training set of the i^{th} preset machine learning model, wherein 1 ≤ i ≤ N, 1 ≤ j ≤ M, and i and j are both positive integers.

3. The odor detecting method according to claim 1 or 2, **characterized in that** the determining odor properties of the gas in the refrigerating appliance according to the odor properties in the N odor detecting results comprise any of the following:
using the odor property with a largest proportion as the odor property of the gas in the refrigerating appliance according to a proportion of each odor property in the N odor detecting results;
taking a median of the odor properties in the N odor detecting results and using the odor property corresponding to the median as the odor property of the gas in the refrigerating appliance; and
taking an average of the odor properties in the N odor detecting results, and using the odor property corresponding to the average as the odor property of the gas in the refrigerating appliance.

4. The odor detecting method according to claim 3, **characterized in that** the odor properties comprise at least pleasant and unpleasant.

5. The odor detecting method according to claim 4, **characterized in that**
when the median of the odor properties in the N odor detecting results is taken, the odor property close to the median and closest to the unpleasant is used as the odor property of the gas in the refrigerating appliance if the median has no corresponding odor property; or
when the average of the odor properties in the N odor detecting results is taken, the odor property close to the average and closest to the unpleasant is used as the odor property of the gas in the refrigerating appliance if the average has no corresponding odor property.

6. The odor detecting method according to any of the preceding claims, **characterized in that** the N preset machine learning models are respectively constructed by using different algorithms, or the N preset machine learning models are constructed by using the same algorithm, or at least two of the N preset machine learning models are constructed by using different algorithms.

7. The odor detecting method according to claim 6, **characterized in that** the algorithm comprises any of the following: a multi-layer neural network classifier, a K-nearest neighbor classifier, a decision tree algorithm, and a support vector machine classifier.

8. The odor detecting method according to any of the preceding claims, **characterized in that** at least one of the N preset machine learning models comprises a plurality of sub-models, the plurality of sub-models are constructed by using different algorithms, and the odor detecting results of the preset machine learning model comprising the plurality of sub-models are obtained based on the odor detecting results obtained by the plurality of sub-models.

9. The odor detecting method according to claim 1, **characterized in that** after the acquiring environmental data of gas in a refrigerating appliance, the method further comprises:
preprocessing the environmental data, wherein a preprocessing method used for data in at least one dimension in the environmental data is different from a preprocessing method used for data in other dimensions.

10. The odor detecting method according to claim 9, **characterized in that** the preprocessing the environmental data comprises:
performing parameter scaling processing on the environmental data to unify the data in different dimensions into a data range of the same dimension, wherein a parameter scaling method used for the data in the at least one dimension is different from a parameter scaling method used for the data in the other dimensions; and
standardizing the processed environmental data so that the environmental data satisfies normal distribution, and extracting a feature of the processed environmental data to obtain the preprocessed environmental data.

11. The odor detecting method according to claim 10, **characterized in that** the environmental data comprises gas data, temperature data, and humidity data, and the performing parameter scaling processing on the environmental data to unify the data in different dimensions into a data range of the same dimension comprises:
taking a logarithm of a value of the gas data to nonlinearly compress the value range of the gas data into a preset value interval; and
performing maximum minimization on the temperature data, the humidity data, and the gas data after taking the logarithm, to obtain the processed environmental data.

12. The odor detecting method according to claim 10 or 11, **characterized in that** before the standardizing the processed environmental data, the preprocessing the environmental data further comprises:
performing data smoothing on the processed environmental data.

13. A refrigerating appliance, **characterized by** comprising:
a gas detector, configured to collect environmental data of gas in the refrigerating appliance; and
a control module, configured to communicate with the gas detector to receive the environmental data, wherein the control module performs the odor detecting method according to any of claims 1 to 12.

14. The refrigerating appliance according to claim 13, **characterized by** further comprising a deodorization module, wherein
the control module is further configured to generate a control instruction according to a current odor property in the refrigerating appliance; and
the deodorization module adjusts an operating parameter according to the control instruction in response to the received control instruction.
